Europäisches Patentamt

European Patent Office

Office européen des brevets

(11) Publication number: **0 394 045**
**A2**

# EUROPEAN PATENT APPLICATION

(21) Application number: **90304223.2**

(22) Date of filing: **19.04.90**

(51) Int. Cl.5: **A61K 31/675, A61K 9/14,**
**A61K 47/18**

(30) Priority: **20.04.89 US 340978**

(43) Date of publication of application:
**24.10.90 Bulletin 90/43**

(84) Designated Contracting States:
**AT BE CH DE DK ES FR GB GR IT LI LU NL SE**

(71) Applicant: **ERBAMONT, INC.**
**c/o Adria Laboratories, 7001 Post Road**
**Dublin, Ohio 43017(US)**

(72) Inventor: **Palepu, Nageswara R.**
**7775 Sanbrooke Road**
**Dublin, Ohio 43017(US)**
Inventor: **Hutt, Julie A.**
**3730 Baybridge Lane**
**Dublin, Ohio 43017(US)**

(74) Representative: **Deans, Michael John Percy et al**
**Lloyd Wise, Tregear & CO. Norman House**
**105-109 Strand**
**London WC2R OAE(GB)**

(54) **Cyclophosphamide lyophilizates.**

(57) A stable rapidly dissolving lyophilized composition of cyclophosphamide and alanine is described which contains an amount of water which ranges from approximately 0 parts to about 10 parts per 100 parts of cyclophosphamide. The composition is preferably prepared from a solution containing at least 1.7% (W/V) weight of alanine and having a pH in the range of 5 to 7. The composition does not require rehydration prior to storage.

## CYCLOPHOSPHAMIDE LYOPHILIZATES

The present invention relates to a novel lyophilized composition containing cyclophosphamide.

U.S. Patent 4,537,883 to Alexander et al. (Mead Johnson & Co.) discloses various lyophilizates of cyclophosphamide. These lyophilizates are prepared by lyophilizing a solution of cyclophosphamide and one or more excipients and re-hydrating the product such that it contains about 4% moisture. The patent is based upon a comparative study of lyophilizate cakes and the dissolution time for lyophilizates of cyclophosphamide prepared using a number of excipients. The study concludes that the lyophilizate prepared with mannitol gives a better cake and faster dissolution time than the lyophilizates prepared with other excipients. The patent also teaches that the lyophilized cyclophosphamide-mannitol composition exhibits better thermal stability if it contains an equimolar amount of water based on cyclophosphamide. The preferred lyophilizate contains 20 parts cyclophosphamide, 1.25 to 2 parts water and 10 to 85 parts mannitol. Among the excipients evaluated in the patent are mannitol, sodium bicarbonate, lactose, polyvinyl pyrrolidone (PVP), arginine, and tartaric acid and combinations of mannitol and various organic acids including the amino acids glycine and arginine as secondary excipients. The lyophilizates illustrated in the patent prepared with the amino acids provided poor cakes which exhibited poor dissolution times.

One problem with the lyophilizates according to U.S. Patent No. 4,537,883 was that they must be rehydrated prior to storage. It was hypothesized that the lyophilizate, prior to rehydration was in an unstable amorphous form. Accordingly, rehydration was required to enable the lyophilizate to be a shelf-stable product.

We have now found that lyophilizates of cyclophosphamide having improved dissolution times and good shelf stability can be obtained using alanine as the excipient. Lyophilizates produced in accordance with the present Invention generally contain about 0 to 10 parts by weight water, and about 50 to 150 parts by weight of alanine per 100 parts by weight cyclophosphamide. The lyophilizates can take the form of either an anhydrous crystalline solid or a crystalline monohydrate solid. The anhydrous form contains between 0 and 0.5 parts water per 100 parts of cyclophosphamide and the monohydrate crystalline form contains between 6.5 and 10 parts water per 100 parts cyclophosphamide. It may further be desirable to rehydrate the lyophilizate prior to storage, although this is not required. When the optional rehydration step is performed, the amount of water in the lyophilizate after rehydration is preferably about equimolar to the amount of cyclophosphamide. In addition to dissolving quickly, these lyophilizates generally experience less than 5% loss in potency when stored at 37°C for a period of six weeks for the monohydrate form, and less than 15% loss in potency for the anhydrous form.

As will become clear from the detailed description hereinbelow, embodiments of our composition provide a good cake, dissolve rapidly when reconstituted with water and provide good shelf stability. The lyophilizate is crystalline in nature and is less susceptible to coagulation during storage. The lyophilized cyclophosphamide does not need to be rehydrated prior to storage.

The invention is hereinafter more particularly described by way of example only.

Previously, as disclosed in U.S. Patent 4,537,883, unsatisfactory lyophilizates have been obtained using the amino acids arginine and glycine as excipients. These lyophilizates provided poor quality cakes which were slow to dissolve. Further, the cakes had to be rehydrated prior to storage. We have now found that lyophilizates of cyclophosphamide and the amino acid alanine having good shelf life and minimum dissolution time can be obtained without requiring rehydration.

Conventional lyophilization techniques that can be used in practice of the present invention include the methods described in U.S. Patent 4,537,883, among other methods known to those skilled in the art. The conditions employed in the Examples which follow are one example of those which can be used.

It is hypothesized that when utilizing alanine as an excipient, upon lyophilization, the lyophilizate is crystalline and anhydrous in form. This material preferably includes about 0 to 0.5 parts water and about 50 to 150 parts alanine per 100 parts of cyclophosphamide. The amount of alanine in solution typically ranges between 1.7 and 5% and the pH of the composition typically ranges between 5 and 7. By comparison, lyophilized compounds produced from excipients known in the art such as mannitol, arginine and glycine, are amorphous in structure. To produce a self stable product, these amorphous materials are rehydrated whereupon they convert to the crystalline monohydrate form. Alanine is unusual as the resultant lyophilized material is crystalline in form and not amorphous.

Following lyophilization, the lyophilizate is preferably rehydrated. After rehydration, the water content is increased to about 6.5 to 10 parts per 100 parts of cyclophosphamide. In between the range of 0.5 and 6.5 parts water per 100 parts of cyclophosphamide, the lyophilizate takes the form of an amorphous solid. The amorphous solid is unstable in the sense that it can lose up to 80% of its potency when stored at 37°C for a period of six weeks. Rehydration can be accomplished by aspirating water into the vial containing the lyophilizate using an ultrasonic spray nozzle which delivers a predetermined amount of water or by placing the vial in a humidity chamber having a relative humidity exceeding 85%. The rehydration step converts the crystalline lyophilizate from an anhydrous form to a monohydrate form. Both the crystalline anhydrous form and the crystalline monohydrate form are shelf stable for extended period of time, with the monohydrate form being the more stable of the two. When the lyophilizate is rehydrated, it is preferred that it contains an amount of water which is about equimolar to the amount of cyclophosphamide to fully convert the lyophilizate to a crystalline monohydrate form.

Our lyophilizates are preferably prepared by lyophilizing solutions containing about 1 to 4% (W/V) cyclophosphamide. Conventional lyophilization conditions can be employed to lyophilize the compositions.

The effect of alanine, the concentration of the pre-lyophilized solution, and the water content of the lyophilizate on the stability of cyclophosphamide lyophilizates are illustrated in the following non-limiting examples.

## Example 1

Lyophilizates of cyclophosphamide and alanine were prepared as follows:

Solutions (5 ml) containing 2% (W/V) cyclophosphamide and alanine in the percentages indicated in the following table were placed in 10cc vials. The vials were frozen in a lyophilization chamber for about 12 hours at a shelf temperature of -26.C. The chamber was then evacuated to a pressure of about 100 millitorr. The samples were maintained in the chamber at a shelf temperature of 0°C for 16 hours and 25°C for 8 hours. After lyophilization was completed, the samples were rehydrated by placing them in an 85% humidity chamber and monitoring the weight gain. The amount of water in the lyophilizate expressed as a percent of the total composition and as a percentage of cyclophosphamide (CP) is shown in the table.

The samples were next subjected to an aging study wherein they were first assayed by HPLC, then placed in an oven at 37°C for the periods indicated and finally reassayed. The loss in potency expressed as a percentage loss based on the initial assay is shown in the table.

Table

| Sample No. | Excipient | % | pH | % Water[1] | % Water-CP[2] | Age (wks) | Loss in Potency |
|---|---|---|---|---|---|---|---|
| 1 | Alanine | 3 | | 1.1 | 3.0 | 6 | 85% |
| 2 | Alanine | 3 | | 3.7-4.8 | 9.6-12.5 | 6 | less than 5% |

1> percentage of total vial
2> percentage based on cyclophosphamide

## Example 2

Lyophilizates of cyclophosphamide and amino acids, more particularly glycine, DL-valine, DL-serine and DL-alanine were prepared using the method of Example 1. The resulting lyophilizates had good cake characteristics and dissolved quickly on reconstitution to a pH near 6. For each of the different amino acid excipients, some vials were rehydrated while others were not. The samples were subjected to an aging study wherein they were assayed by HPLC after 6 weeks of storage at 37%C. All of the rehydrated vials showed excellent stability. However, with respect to the vials which were not rehydrated, the samples containing either glycine, DL-valine or DL-serine excipients had less than 20% of active pharmaceutical remaining, indicating that these materials needed to be rehydrated to be shelf stable. The sample which utilized alanine as the excipient maintained 84% of its original amount, indicating that the non-rehydrated lyophilizate could be used as a shelf stable product. Six and eighteen-week stability data for samples which utilized alanine as an excipient are set forth in Table 2.

3

Table 2

| Cyclophosphamide/DL-Alanine Stability at 37°C | | | |
|---|---|---|---|
| | | Stability 37°C | |
| Sample | Weight Gain during rehy-l dration (mg) | 6 wks | 18 wks |
| 94 mg cyclophosphamide/ 150 mg DL-Alanine | Not rehydrated 5.6 | 84% 102% | -- 98% |

Example 3

A lyophilizate of cyclophosphamide was produced by providing a solution containing 20mg/ml cyclophosphamide and 30mg/ml of 3%-DL-alanine, and 5ml of the solution was inserted into several 10 ml vials. The vials were immediately frozen at a temperature of approximately -15%C and lyophilized according to standard procedure. The vial was tested for stability at 37°C and assayed after 6 and 12 weeks. After 6 weeks, the vial contained 85% of the initial material. After 12 weeks, the vial contained 90% of the original material.

The results of the examples show that a cyclophosphamide lyophilizate can be produced without requiring a rehydration step when alanine is utilized as an excipient. The studies further show that the non-rehydrated samples are shelf stable for at least 6, and in some instances, 12 weeks at 37°C. Based upon X-ray diffraction data taken from the samples, it is hypothesized that the unexpected stability of the lyophilizates is directly attributable to the crystalline anhydrous form. The study further shows that additional shelf stability may be obtained by re-hydrating the lyophilizate prior to storage to convert the crystalline anhydrous form to a crystalline monohydrate form.

Example 4

Cyclophosphamide lyophilizate samples were produced by lyophilizing 5ml aliquots of solutions containing 33.3 mg/ml cyclophosphamide and either 17.7 mg/ml, 35.4 mg/ml or 53.1 mg/ml of L-alanine. The concentrations of L-alanine added were selected to provide samples having weight ratios. of cyclophosphamide to L-alanine of approximately 1:0.5, 1:1 and 1:1.5 respectively. Two samples at each weight ratio were produced. The solutions were frozen at -35°C. Drying was performed by utilizing a primary drying step at -5°C for 24 hours followed by a secondary drying step at 25°C for 8 hours. The samples were analyzed by X-ray diffraction and differential scanning calorimetry. The 1:0.5 and 1:1 lyophilizates were determined to be crystalline anhydrous solids whereas the 1:1.5 lyophilizates were determined to be an amorphous solid. The materials were stored at 37°C for two months, and the samples were then analyzed for stability. The 1:0.5 samples had an average of 92% material remaining and the 1:1 samples had an average of 90% material remaining. One vial of the 1:1.5 sample contained 90% of its original material while the other vial contained 18% of its original material.

**Claims**

1. A lyophilized composition containing cyclophosphamide and the amino acid alanine as excipient.

2. A lyophilized cyclophosphamide composition characterised in comprising cyclophosphamide, alanine and water wherein the amount of water ranges from approximately 0 parts to about 0.5 parts or from about 6.5 parts to about 10 parts per 100 parts by weight of cyclophosphamide, and wherein alanine is present in an amount of about 50 to 150 parts per 100 parts by weight of cyclophosphamide.

3. A composition according to Claims 1 or 2, further characterised in that said composition comprises a lyophilizate of a solution of cyclophosphamide containing at least 1.7% by weight of alanine, without rehydration.

4. A composition according to Claim 3, further characterised in that said lyophilizate comprises a crystalline anhydrous solid wherein the amount of water ranges from approximately 0 parts to about 0.5 parts per 100 parts by weight of cyclophosphamide.

5. A composition according to Claims 1 or 2, wherein water is present in said lyophilized composition in an amount of about 6.5 to 10 parts by weight, and alanine is present in an amount of about 50 to 150 parts by weight per 100 parts by weight of cyclophosphamide, the composition being further characterised in comprising a rehydrated lyophilizate of a solution of cyclophosphamide containing at least about 1.7% by weight of alanine.

6. A composition according to Claim 5, further characterised in that said lyophilizate comprises a crystalline monohydrate solid.

7. A shelf stable lyophilized composition comprising cyclophosphamide, and having a water content which ranges from approximately 0 parts to 0.5 parts per 100 parts cyclophosphamide, said composition essentially comprising a stored lyophilizate of a solution of cyclophosphamide and alanine.

8. A composition according to Claim 7, further characterised in that said composition comprises a crystalline anhydrous solid.

9. A composition according to any preceding claim, further characterised in that said composition contains about 1.7% to 5% of alanine.

10. A composition according to any preceding claim, further characterised in said composition possessing a pH in the range of about 5.0 to 7.0.

11. A process for preparing a lyophilized composition of cyclophosphamide, characterised in comprising the step of lyophilizing a solution of cyclophosphamide containing at least about 1.7% by weight of alanine to produce a lyophilizate which contains an amount of water ranging from about 0 parts to about 0.5 parts per 100 parts by weight of cyclophosphamide.

12. A process according to Claim 11, characterised in further comprising the additional step of rehydrating said lyophilizate to increase the amount of water in said composition to between about 6.5 parts and about 10 parts per 100 parts by weight of cyclophosphamide.

13. A process according to Claims 11 or 12, further characterised in that said composition contains about 1.7 to 5% alanine.

14. A process according to any of Claims 11, 12 or 13, further characterised in that said composition possesses a pH of about 5-7.